# EUROPÄISCHE PATENTSCHRIFT

(11) **EP 0 705 103 B1**
(45) Veröffentlichungstag und Bekanntmachung des Hinweises auf die Patenterteilung: **28.12.2005**
(21) Anmeldenummer: 95911144.4
(22) Anmeldetag: 16.03.1995
(51) Int. Cl.: A61K 31/705, A61P 7/00, A61P 9/10

(54) **VERWENDUNG VON NOTOGINSENOSID R1 ZUR BEHANDLUNG VON ENDOTOXINSCHOCK ODER HERZERKRANKUNGEN**
USE OF NOTOGINSENOSIDE R1 FOR THE TREATMENT OF ENDOTOXIN SHOCK OR HEART DISEASES
UTILISATION DE LA NOTOGINESENOSIDE R1 POUR LE TRAITEMENT DU CHOC D'ENDOTOXINE OU DES MALADIES CARDIAQUES

(30) Priorität: 16.03.1994 AT 56194
(43) Veröffentlichungstag der Anmeldung: 10.04.1996
(73) Patentinhaber: BERBI Gesellschaft m.b.H., A-1010 Wien (AT)
(72) Erfinder: BINDER, Bernd, A-1090 Wien (AT); ZHANG, Weijian, A-1090 Wien (AT); WOJTA, Johann, A-1090 Wien (AT)
(74) Vertreter: Patentanwälte BARGER, PISO & PARTNER
(86) Internationale Anmeldenummer: PCT/AT1995/000049
(87) Internationale Veröffentlichungsnummer: WO 1995/024905

(56) Entgegenhaltungen:
- PLANTA MEDICA, Bd. 55, Nr. 1, Februar 1989 Seiten 18-21, H. MATSUDA ET AL. 'Studies of panax japonicus fibrinolysis'
- CHEM. PHARM. BULL., Bd. 34, Nr. 5, Mai 1986 Seiten 2100-2104, H. MATSUDA ET AL. 'Pharmacological study on panax ginseng C.A. Meyer IV. Effects of red ginseng on experimental disseminated intravascular coagulation (3) effect of ginsenoside-Ro on the blood coagulative and fibrinolytic system'
- CHEM. PHARM. BULL., Bd. 34, Nr. 3, März 1986 Seiten 1153-1157, H. MATSUDA ET AL. ' Pharmacological study on panax ginseng C.A. Meyer. III. effect of red ginseng on experimental disseminated intravasular coagulation. (2) effects of ginsenosides on blood coagulative and fibrinolytic systems'
- CHEM. PHARM. BULL., Bd. 39, Nr. 5, 1991 Seiten 1185-1188, YOSHIKAWA K. ET AL. 'Structure of two new fibrinolytic saponins from the seed of Luffa cylindrica ROEM'
- ANNALS OF HEMATOLOGY, Bd. 70, Nr. Suppl., 1995 Seite A92 W.J. ZHANG ET AL. 'Astragaloside counteracts endotoxin stimulated induction of both plasminogen activator inhibitor-1 and tissue factor in cultured human endothelial cells'
- FIBRINOLYSIS, Bd. 8, Nr. Suppl. 1, 1994 Seite 119 W.J. ZHANG ET AL. ' Notoginsenoside R1 counteracts the induction of plasminogen activator inhibitor-1 and tissue factor in response to endotoxin stimulation in vitro and in vivo'
- FIBRINOLYSIS, Bd. 8, Nr. Suppl. 1, 1994 Seite 150 ZHANG WJ 'Effect of Notoginsenoside R1 on the synthesis of components of the fibrinolytic system in cultured human pulmonary artery vascular cells'

## Beschreibung

### Hintergrund

Das fibrinolytische System dient als basaler Abwehrmechanismus, der die Ablagerung von Fibrin sowohl im vaskulären, als auch in extravaskulären Systemen kontrolliert. Das richtige Funktionieren des fibrinolytischen Systems ist notwendig, um einerseits hämorrhagische und andererseits thrombotische Phänomene zu verhindern, aber auch um die Bildung von interstitialen Fibrinablagerungen und darauffolgende Narbenbildung zu verhindern. Man nimmt an, daß der Gewebeplasminogenaktivator (t-PA) eine wichtige Rolle in der Auslösung der (extrinsischen) fibrinolytischen Kaskade durch die Umwandlung des Zymogens Plasminogen in das aktive Plasmin, das Fibrin abbaut, spielt. Man nimmt weiters an, daß daher die fibrinolytische Kapazität des Plasmas signifikant von der Konzentration des zirkulierenden t-PA abhängt. Der t-PA im Plasma stammt wahrscheinlich hauptsächlich aus der Gefäßwand, wo er in der Endothelzelle lokalisiert ist. Weiters spielt der Urokinase Plasminogenaktivator (u-PA) eine Rolle in der Gesamt-Fibrinolyse. Man nimmt an, daß dieser Plasminogenaktivator auch - zumindest teilweise - aus der Gefäßwand stammt. Der Hauptinhibitor der Fibrinolyse, Plasminogenaktivatorinhibitor 1 (PAI-1) wird auch von Endothelzellen synthetisiert und es existieren Daten, die zeigen, daß das relative Mengenverhältnis zwischen PAs und PAI-1 wichtig ist für die fibrinolytische Kapazität und damit für die Verhinderung von thrombotischen Abläufen wie z.B. beim Myokardinfarkt. Die pharmakologische Regulierung der Synthese von t-PA, u-PA und PAI-1 ist daher von Nutzen, um eine unzureichende, endogene Fibrinolyse zu steigern.

Da sowohl t-PA, als auch PAI-1 von Endothelzellen produziert werden, stellt die Regulation ihrer Synthese und Sekretion auf dem Niveau der Endothelzelle einen raschen und direkten Weg dar, das fibrinolytische Potential des Blutes zu beeinflussen. Kürzlich durchgeführte Studien haben gezeigt, daß die Produktion von Plasminogenaktivatoren und Inhibitoren in verschiedenen Zelltypen durch eine Reihe von Faktoren reguliert wird: Die Synthese von t-PA in Endothelzellen wird durch eine Vielzahl von Stimuli wie z.B. Thrombin, Histamin, Butyrat, Retinolsäure und Tumorpromoteren, wie z.B. Phorbol-12-Myristat-13-Acetat (PMA) gesteigert. Faktoren, die die Expression von PAI-1 regulieren, schließen Lipopolysaccharide, Thrombin, Interleukin 1 (IL-1), Tumornekrosefaktor α (TNF α), Transforming Growth Factor β (TGF β), Basic Fibroblast Growth Factor (BFGF) und Endothelial cell Growth Supplement in Kombination mit Heparin ein. Keine der oben angeführten Substanzen konnte allerdings in vivo erfolgreich angewendet werden.

Die bakterielle Sepsis, ausgelöst durch die Freisetzung bakteriellen Endotoxins (LPS), ist ein lebensbedrohender Zustand, bei dem durch LPS ausgelöste Veränderungen in der Gerinnung und Fibrinolyse intravaskuläre Gerinnselbildung und in der Folge Organversagen verursachen. Man nimmt an, daß dabei LPS auf Endothelzellen einwirkt, in dem es deren Expression von Tissue Factor (TF) und PAI-1 steigert. Zur Zeit ist keine ausreichende direkte Behandlung von Patienten, die an einer durch LPS ausgelösten intravaskulären Gerinnung leiden, möglich und Maßnahmen zur Behandlung der durch LPS ausgelösten Symptome, wie z.B. Hyperkoagulation sind einerseits beschränkt auf Heparin und andererseits auf die Behandlung der verursachenden bakteriellen Sepsis mit Antibiotika. In China wurden chinesische Kräuterdrogen wie Panax Notoginseng oder Astragalose seit Jahrtausenden von traditionellen chinesischen Ärzten zur Schmerzlinderung und zur Behandlung von Stase und kardiovaskulären Erkrankungen verwendet.

Tatsächlich wird zum Beispiel in L. Zechmeister, Progress in the Chemistry of Organic Natural Products, Vol. 46, Springer-Verlag, Wien New York 1984, in dem Kapitel über "Saponins of Ginseng and Related Plants" auf die Eigenschaft von Panax notoginseng als Tonikum, Hämostaticum, Coronartherapeuticum und Antihemorrhagicum hingewiesen. Ebenso wird in Chemical Abstracts 119, 85 683 auf eine antithrombotische Wirkung von Panax notoginseng und in dem Japanischen Patent Abstracts JP Kokai Nr. 55-127 317 auf die antifibrinolytische Wirkung und JP Kokai Nr. 63-198 609 auf die durchblutungsfördernde Wirkung von Panax notoginseng-Präparaten hingewiesen.

H. MATSUDA ET AL.: "Studies of panax japonicus fibrinolysis" PLANTA MEDICA, Bd. 55, Nr. 1, Seiten 18-21, beschreiben eine aktivierende Wirkung von triterpenen Saponinen auf die fibrinolytische Aktivität der Ratte und zur Behandlung der endotoxin induzierten disseminierten intravasalen Gerinnung. Es wird jedoch nur eine globale Aktivierung der Fibrinolyse ohne Angabe der beeinflussten Moleküle beschrieben.

In H. MATSUDA ET AL. "Pharmacological study on panax ginseng C.A. Meyer IV. effects of red ginseng on experimental disseminated intravascular coagulation (3) effect of ginsenoide-Ro on the blood coagulative and fibrinolytic systems" CHEM. PHARM. BULL. Bd. 34, Nr. 5, Seiten 2100-2104, werden ebenfalls die Effekte von rotem Ginseng auf Gerinnung und Fibrinolyse bei Ratten beschrieben allerdings ohne nähere Angaben eines molekularen Mechanismus für den globalen Effekt.

In einer weiteren Veröffentlichung H. MATSUDA ET AL. "Pharmacological study on panax ginseng C.A. Meyer III. effect of red ginseng on experimental disseminated intravascular coagulation. (2) effects of ginsenoides on blood coagulative and fibrinolytic systems" CHEM. PHARM. BULL., Bd. 34, Nr. 3, Seiten 1153-1157, wird für die stimulierung der fibrinolytischen Aktivität die Urokinase verantworltich gefunden. Auch YOSHIKAWA ET AL. "Structure of two new fibrinolytic saponies from the seed of Luffa cylindra ROEM" CHEM. PHARM. BULL., Bd. 39, Seiten 1185-1188 beschreiben zwar die Struktur von zwei neuen "fibrinolytischen" Saponinen, nicht jedoch auf molekularer Ebene beim Menschen.

BIOSIS PREV199294099689 Abstract - AMERICAN JOURNAL OF CHINESE MEDICINE, Bd. 20, Nr. 2, 1992 Seiten 167-173, KOYAMA N; MORISAKI N; SAITO Y; YOSHIDA 5 "Inhibitory effect of ginsenosides on migration of arterial smooth muscle cells" bezieht sich auf die Verhinderung der Lipidperoxidation und damit auf einen völlig unterschiedlichen Mechanismus der Wirkung von Panax Notoginseng ungereinigten Extrakten.

### XP001059665 - ZHONGUA YAOLI XUEBAO - ACTA PHARMACOLOGICA

SINICA, SHANGHAI, CN, Bd. 11, Nr. 1, 1990 Seiten 26 - 29, Li X; CHEN J-X; SUN J-J "Protective effects of panax notoginseng saponins on experimental myocardial injury induced by ischemia and reoerfusion in rat" bezieht sich auf die Migration von glatten Muskelzellen, einen weiteren möglichen

Wirkmechanismus.

### Generelle Beschreibung der Erfindung

Es wird hier zum ersten Mal gezeigt, dass die Verwendung des Triterpensaponins Notoginsenosid R1 (NR1) die fibrinolytische Kapazität durch Steigerung des t-PA und durch Senkung des PAI-1 beim Menschen und der Maus steigert und zur direkten Hemmung der LPS-Effekte in Bezug auf PAI-1 und Gewebefaktor Anstieg führt. Gegenstand dieser Erfindung ist die Verwendung von Notoginsenosid R1 (NR1) zur Herstellung eines Arzneimittels zur Behandlung von Endotoxin-Schock. Das Arzneimittel kann entweder parenteral oder oral in Form von Lösungen oder Tabletten oder Kapseln verabreicht werden. Insbesondere betrifft die Erfindung die Verwendung von Notoginsenosid R1 (NR1) zur Herstellung eines Arzneimittels zur Behandlung von koronarer Herzerkrankung, peripherer Arterienerkrankung sowie Myocardinfarkt und Angina pectoris.

### Beispiele

In allen Beispielen wurden die folgenden Materialien und Methoden benutzt.

Chemisch-reines Notoginsenosid R1 (NR1) wurde vom National Institute for the Control of Pharmaceutical and Biological Products (Beijing, China) gekauft. NR1 sind Substanzen mit der folgenden Formel: 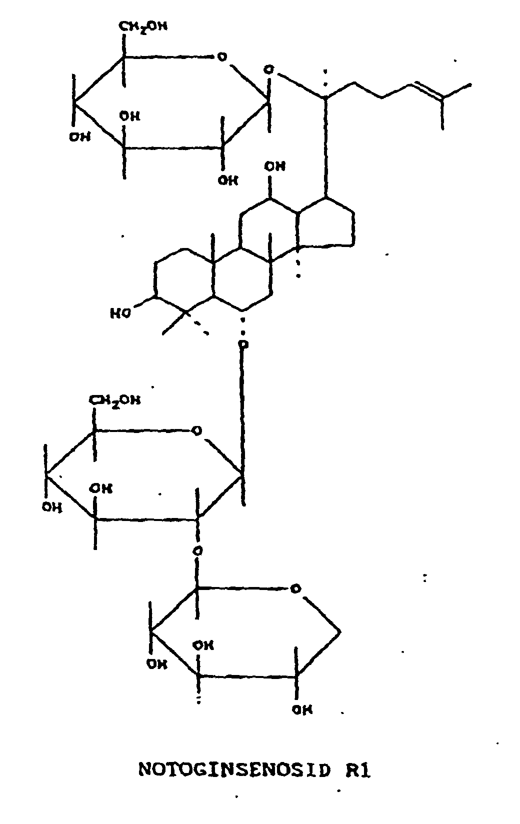 NR1 wurde in Inkubationsmedium aufgelöst und verdünnt, um eine finale Konzentration von 0,01 bis 100 µg/ml zu erreichen. Lipopolysaccharid (Escherechia coli lipopolysaccharid, Serotyp 026:B6 präpariert durch Phenolextraktion wurde von Sigma (St. Louis, MO, USA) bezogen. Eine Lösung mit der Konzentration von 1 mg/ml in destilliertem Wasser wurde bei -70°C aufbewahrt. Morpholinopropansulfonsäure (Serva, Deutschland), Guanidin Thiozyanat (Fluka, Schweiz), Piperazin-N,N'-bis[2-Ethan-Sulfonsäure] (PIPES; Sigma), Seakem LE Agarose (FMC Bioproducts, ME, USA), dCTP [Aloha-32P] (ICN Radiochemicals, CA, USA) wurden von den erwähnten Firmen bezogen. Die übrigen Materialien, die in den Methoden beschrieben werden, sind im Detail in den entsprechenden Zitaten angeführt.

### Zellkultur

Endothelzellen wurden aus frischen humanen Nabelschnurvenen mit Kollagenase (Sigma) isoliert, ähnlich einem Protokoll, das von Jaffe et al., J Clin Invest 1973; 52: 2745-56, beschrieben wurde. Zellen aus 4-6 Nabelschnüren wurden gepoolt und in 75 cm² Zellkulturflaschen (Costar, MA, USA) ausgesät, die mit 1% Gelatine aus Kälberhaut (Sigma) beschichtet waren. Die Zellen wurden bis zur Konfluenz bei 37°C in einer wasserdampfgesättigten Athmosphäre aus 95% Luft und 5% CO₂ in Medium 199 (Sigma) versetzt mit 20% hitzeinaktivierten Supplementierten Kälberserum (SCS; Hyclone, UT, USA), 100 µg/ml Streptomycin, 100 IU/ml Penizillin, 250 ng/ml Fungizon, 1 mM Glutamin (JHR Biosciences, KS, USA) 2 IU/ml Heparin (Liquemin Roche; Hoffmann La Roche, Schweiz), 50 µg/ml ECGS (Technoclone, Austria) kultiviert. Der Endothelcharakter der Zellen wurde durch ihre typische Pflastersteinmorphologie, durch positive Immunfluoreszens mit Anti-Von willebrandt Faktor VIII Antikörpern und durch Aufnahme von azetyllierten Low Density Lipoprotein (LDL) charakterisiert. Primärkulturen wurden zum Zeitpunkt der Konfluenz mit 0,05% Trypsin/0,02% EDTA (JRH Biosciences) geerntet und in einer Split-ratio von 1:3 in 75cm² Zellkulturflaschen ausgesät. Subkonfluente Zellen wurden unter den selben Bedingungen bis zum Erreichen der Konfluenz kultiviert und wurden während des exponentiellen Zellwachstums mit Trypsin/EDTA geerntet und in 1 ml Portionen in Medium 199 mit 10% Dimethylsulfoxid (DMSO) im flüssigen Stickstoff eingefroren. Für die Experimente wurden die Zellen bei 37°C aufgetaut und in 6 well Platten (Durchmesser 3,5 cm; Costar) in Medium 199 versetzt mit SCS, ECGS und Heparin in den oben angeführten Konzentrationen bis zum Erreichen der Konfluenz kultiviert. In allen Experimenten wurden Zellen zwischen der 2. und 3. Passage verwendet. Die Zellen wurden immer am Tag vor den Experimenten mit frischem Medium gefüttert. Alle in der Zellkultur verwendeten Materialien waren wie durch den Coatest Endotoxin Kit (Kabi Vitrum, Schweden) bestimmt frei von Endotoxin (Nachweisgrenze des Test 5 pg/ml).

Herstellung des konditionierten Mediums (CM) und der extrazellulären Matrix (ECM)

Konfluente Kulturen wurden zwei Mal mit Hank's Balanced Salt Solution (HBSS; Sigma) gewaschen und bei 37°C mit 1 ml/Napf von Medium 199 versetzt mit 1,25% SCS und 50 µg/ml ECGS und mit NR1 oder AS IV in den angeführten Konzentrationen inkubiert. Nach der Inkubation wurde der Zellkulturüberstand gesammelt und nach der Zentrifugation - um Zellbruchstücke zu entfernen - bei -70°C bis zum Gebrauch gelagert. Die Gesamtzellzahl der entsprechenden Kulturen wurde nach Trypsinisierung mit der Zählkammer bestimmt. ECM von diesen oder ähnlich behandelten Kulturen wurde nach der Methode von Mimuro et al., Blood 1987; 70: 721-28 präpariert. Die Monolayer wurden 3 Mal mit kalter Phosphat gepufferter Kochsalzlösung (PBS: 0,01 M Natriumphosphat, 0,14 M NaCl, pH 7,4) gewaschen und die zellulären Bestandteile wurden durch 10 minütige Inkubation bei 37°C mit PBS versetzt mit 0,5% Triton X100 extrahiert. Die Platten wurden ein weiteres Mal mit destilliertem Wasser gewaschen um verbleibende zelluläre Bestandteile zu entfernen und anschließend durch mikroskopische Untersuchung auf das Vorhandensein von Zellbruchstücken hin untersucht. Diese Extraktionsmethode entfernte sichtbare Zellbestandteile von den Platten vollständig und die ECM wurde durch Abschaben in 1 ml PBS versetzt mit 0,1% SDS nach 30 minütiger Inkubation bei 37°C extrahiert. Die Extrakte wurden über Nacht bei 4°C gegen PBS dialysiert.

Tests für tPA Antigen, uPA Antigen, PAI-1 Antigen, PAI-1 Aktivität und tPA PAI-1 Komplexen im CM, in der ECM und im Plasma

tPA Antigen, uPA Antigen, PAI-1 Antigen und die Konzentration von tPA PAI-1 Komplexen wurde mit spezifischen kommerziell erhältlichen Enzyme Linked Immunosorbent Assays (ELISAs) (Technoclone) laut den vom Hersteller beigefügten Anleitungen bestimmt. Die Testbereiche für diese Tests liegen für tPA zwischen 0,3 und 2,5 ng/ml für uPA zwischen 0,6 und 10 ng/ml, für PAI zwischen 1,0 und 30 ng/ml und für tPA PAI-1 Komplexe zwischen 0,2 und 20 ng/ml. Der tPA ELISA bestimmt freien tPA und tPA in Komplex mit PAI-1. Der uPA ELISA bestimmt freien uPA und uPA in Komplex mit PAI-1. Der PAI-1 ELISA mißt freien, komplexierten und latenten PAI-1. Der tPA PAI-1 Komplex ELISA mißt ausschließlich tPA PAI-1 Komplexe. PAI-1 Aktivität im Plasma und im CM wurde mit einem Titrationsassay (Technoclone) laut den vom Hersteller beigefügten Anleitungen bestimmt.

### Bestimmung der funktionellen Aktivität von tPA und PAI-1

Die Aktivität von tPA und PAI-1 wurde nach Natriumdodecylsulfat Polyacrylamid Gelelektrophorese (SDS-PAGE) unter Verwendung von Fibrinautographie (FA) und reverser Fibrinautography (RFA) analysiert. SDS Polyacrylamid Gele und Puffer wurden nach dem Protokoll von Laemmli, Nature 1970; 227: 680-85, hergestellt. FA wurde wie von Granelli-Piperno et al., J Exp Med 1978; 148: 223-34 ausgeführt. 100 µl der entsprechenden Proben wurden auf Gele bestehend aus einem 10 cm langen Trenngel mit 10% Acrylamid und aus einem 2 cm langen Obergel mit 4% Acrylamid aufgetragen und bei Raumtemperatur 16 Stunden lang oder bis die Farbfront den Unterrand des Gels erreichte gefahren. Nach der Elektrophorese wurden die Gele vorerst 90 Minuten in 250 ml Wasser versetzt mit 2,5% Triton X100 (Serva) eingelegt (die Tritonlösung wurde nach 45 Minuten gewechselt), um das SDS zu neutralisieren, dann wurden die Gele auf einen Fibrin-Agar-Indikatorfilm versetzt mit 1,5% Agarose Typ L (Behring, Deutschland), 2 mg/ml plasminogenreichem Fibrinogen (Organon Teknika, Holland) und 0,2 IU/ml bovinem Thrombin (Sigma) gelegt. Die Gele wurden bei 37°C in einer feuchten Kammer inkubiert und zu verschiedenen Zeitpunkten fotografiert. RFA wurde durchgeführt, indem die Gele auf einen Fibrinfilm, der grundsätzlich wie oben beschrieben hergestellt wurde, aber zusätzlich 0,4 IU/ml Urokinase (Technoclone) enthielt, gelegt wurden. Die Quantifizierung der tPA und PAI-1 Aktivität in einer bestimmten Probe wurde erreicht, indem sowohl Lysezonen, als auch Lyse-resistente Zonen auf dem Indikatorfilm fotografiert wurden. Diese Zonen wurden auf Transparentpapier aufgezeichnet und die gezeichneten Areale wurden ausgeschnitten und auf einer analytischen Waage gewogen.

Um den im CM von HUVECs enthaltenen Plasminogenaktivator immunologisch zu identifizieren, wurden Proben des CM bei 4°C für 24 Stunden mit entweder an CNBr aktivierte Sepharose gebundenen monoklonalen Anti-tPA Antikörper (MPW 3 VPA; Technoclone) oder monoklonalen Anti-uPA Antikörper (MPW 5 UK; Technoclone) oder als Kontrolle mit Sepharose 4B (Pharmacia, Schweden) inkubiert. Danach wurde die Sepharose durch Zentrifugation entfernt und 100 µl der entsprechenden Probe wurde mit der oben beschriebenen SDS-PAGE und nachfolgender FA analysiert.

### Präparation der Zell-Lysate und Messung der TF-Aktivität

HUVECs wurden für 6 Stunden bei 37°C in Medium 199 mit LPS und/oder NR1 inkubiert. Die Zellen wurden 3 Mal mit Clotting Puffer (130mM NaCl, 8 mM Na-Barbital und 12 mM Na-Acetat, pH = 7,4) gewaschen und in 300 µl Clotting Puffer durch Abschaben aufgenommen. Die abgeschabten Zellen wurden 3 Mal gefroren und getaut. Die Zell-Lysate wurden in einem 1-Schritt-Clotting Assay auf TF-Aktivität hin untersucht. 100 µl der Zellysate wurden mit 100 µl 20 mM CaCl₂ bei 37°C für 5 Minuten in vorgewärmten Plastikröhrchen in einem Coagulometer (H. Amelung GmbH., Deutschland) inkubiert. Die Gerinnung wurde durch Zugabe von 100 µl vorgewärmten normalen humanem Zitratplasma oder durch Zugabe von Faktor X Mangelplasma (Sigma) ausgelöst. Die TF-Aktivität wurde mit einer Standardkurve (Log-Log Plot), die mit Kaninchenhirn Thromboplastin (Sigma) konstruiert wurde, quantifiziert. 100 mU Aktivität wurden definiert als eine Gerinnungszeit von 20 Sekunden in einem Standardtest mit normalem humanem Plasma. Die beobachtete coagulatorische Aktivität entspricht der TF-Aktivität, da keine procoagulatorische Aktivität der Endothelzellen festgestellt wurde, wenn Faktor X Mangelplasma anstelle von normalem Plasma verwendet wurde.

### Quantifizierung der tPA, PAI-1 und TF mRNA Menge durch Northern Blot Analyse

Die gesamte zelluläre RNA aus Endothelzellen wurde mit Hilfe der von Chomzynski und Sacchi, Anal Biochem 1987; 162: 156-9, beschriebenen sauren Guanidin Thiozyanat-Phenol-Chloroform Extraktion isoliert. Der RNA Niederschlag wurde in 50 µl 0,5% SDS resuspendiert und die Konzentration bei 260 nM bestimmt. Für die Northern Blot Analyse wurden die RNA Proben in einem 1,2%igen Agarosegel elektrophoretisiert und danach die fraktionierte RNA auf eine Duralon-UVTM Membran (Stratagene, CA, USA) durch Kapillarwirkung transferiert. Die RNA Blots wurden in seal-a-meal Säckchen eingeschlossen und in 50 mM PIPES, 100 mM NaCl 50 mM NaPhosphat 1 mM EDTA, versetzt mit 5% SDS für zumindest 3 Stunden bei 57°C prähybridisiert. Der Prähybridisationspuffer wurde verworfen und durch frischen Prähybridisationspuffer versetzt mit 106 CPM/ml der 32 P markierten cDNA-Sonden für entweder humanen tPA, humanen PAI-1, humanen TF oder Ratten-Glyceraldehyd-3-Phosphatdehydrogenase, die als interne Standardsonde verwendet wurde, ersetzt. Die cDNA Fragmente wurden mit einem Random Prime DNA Labelling Kit (Boehringer Mannheim, Deutschland) radioaktiv markiert.

### Tierexperimente

In dieser Studie wurden ausschließlich männliche Balb C Mäuse (18-30 g Körpergewicht) verwendet. Alle Experimente wurden unter Aetherbetäubung durchgeführt. Den Mäusen wurde intravenös über die Schwanzvene LPS (10 ng/g) und/oder NR1 (1 µg/g) in einem Volumen von 5 µg/l injiziert. Zu den angegebenen Zeitpunkten wurden mit Natriumzitrat (0,13 M Endkonzentration) antikoagulierte Blutproben gewonnen. Thrombozytenfreies Plasma wurde durch Zentrifugation bei 2500 g für 30 Minuten bei 4°C gewonnen und bei -70°C bis zur Testung aufbewahrt.

### Verabreichung von NR1-hältigen Extrakten beim Menschen

Es wurde bei 6 freiwilligen gesunden Propanden in einem Zeitraum von 24 Stunden 4 x 100 mg-Äquivalent eines Notoginseng R1 (NR1) enthaltenden Extraktes verabreicht. Es erfolgte eine Blutabnahme unmittelbar vor der ersten Einnahme und unmittelbar nach der letzten Einnahme. In diesen Blutproben wurde entsprechend den oben angegebenen Methoden der Gehalt an Plasminogenaktivator Inhibitor I (PAI-1) Antigen, Gewebeplasminogenaktivator (tPA) Antigen und Urokinase-Plasminogenaktivator (uPA) Antigen bestimmt.

### Statistische Analyse

Die Resultate sind als Mittelwerte ± Standardabweichung angegeben. Ein unpaired Student's t-test wurde verwendet um die Signifikanz festzustellen.

### Beispiel 1: Effekt von NR1 auf die Produktion von tPA, uPA und PAI-1 in kultivierten, humanen Umbilicalvenen Endothelzellen

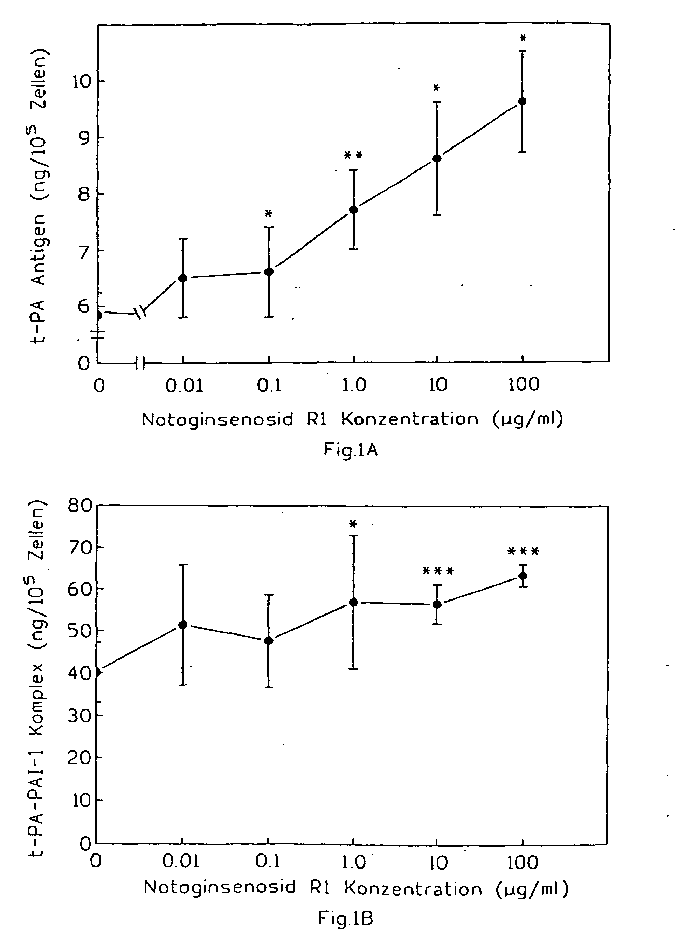

### Effekt von Notoginsenosid R1 (NR1) auf tPA Antigen (Feld A), t-PA PAI-1 Komplexe (Feld B) und PAI-1 Antigen (Feld C) Produktion in kultivierten HUVECs (Fig. 1)

HUVECs wurden für 24 Stunden mit verschiedenen Konzentrationen von NR1 (0,01-100 µg/ml) inkubiert und das CM wurde auf tPA Antigen, PAI-1 Antigen und tPA PAI-1 Komplexe hin wie unter Materialien und Methoden beschrieben, untersucht. Die Resultate sind die Mittelwerte von 3 jeweils in 3-facher Anordnung ausgeführten Experimenten. Die Werte sind als Mittelwerte ± S.D. in Fig. 1 gezeigt. Signifikanzen sind im Vergleich zur Kontrolle angegeben (* p<0,05; ** p<0,01; *** p<0,001). Wie in Fig. 1A gezeigt führte die Behandlung von HUVECs mit steigenden Konzentration von NR1 für 24 Stunden zu einem dosisabhänigen Anstieg des tPA Antigens im CM solcher Art behandelter Zellen: Maximale Effekte wurden mit 100 µg/ml NR1 (100mg/ml NR1: 9,6 ± 0,7 ng/105 Zellen/24h; Kontrolle: 5,8± 0,4 ng/105/24h; n=9, p<0,05) erreicht. Wie in Fig. 1B gezeigt, stiegen auch die tPA PAI-1 Komplexe im CM in ähnlicher Weise in Gegenwart zunehmender Konzentrationen von NR1 an (100 µg/ml NR1: 63,5±2,6 ng/105 Zellen/24h; Kontrolle: 40,2 ± 7ng/105 Zellen/24h; n=9, p<0,01.

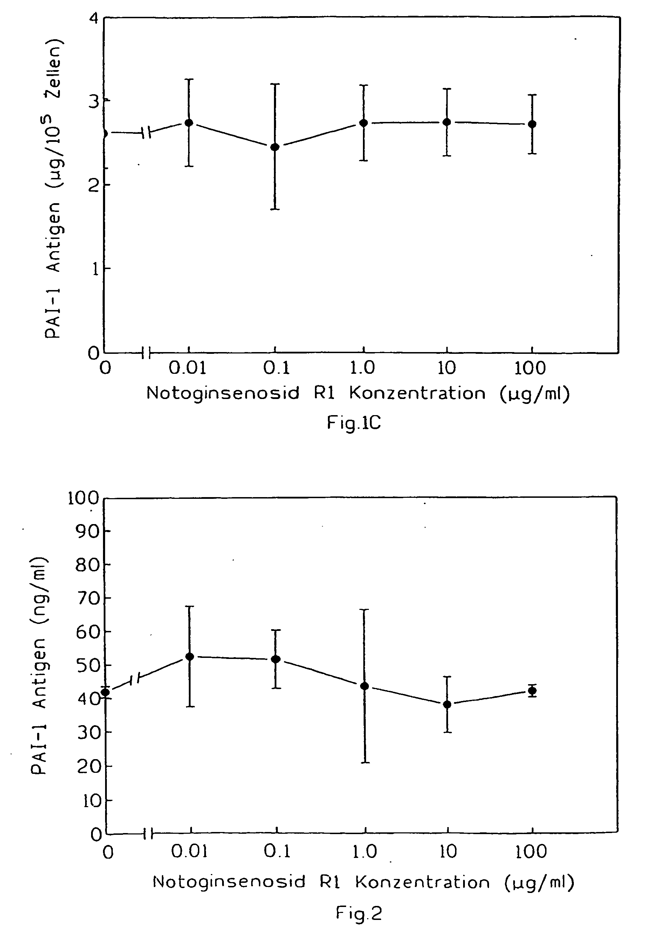

### Effekt von Notoginsenosid (NR1) auf die PAI-1 Antigenproduktion in die ECM von kultivierten HUVECs: (Fig. 2)

HUVECs wurden für 24 Stunden mit verschiedenen Konzentrationen von NR1 (0,01-100µg/ml) inkubiert. Die ECM wurde wie unter Material und Methoden beschrieben, gewonnen und auf PAI-1 Antigen hin untersucht. Die Werte stellen Mittelwerte ± S.D. aus 6 unabhängigen Näpfen dar. PAI-1 Antigen im CM und in der ECM von mit NR1 behandelten HUVECs war nicht signifikant verändert im Vergleich zu den Kontrollen (CM: 100µg NR1/ml: 2,92 ± 0,32 µg/105 Zellen/24h; Kontrolle: 2,78 ± 0,45 µg/105 Zellen/24h; n=9. ECM: 100µg/ml NP1: 42, 55 ± 3,15 ng/ml/24h; Kontrolle: 42,27 ± 1,66 ng/ml/24 h; n=6) (Fig. 1C, Fig. 2).

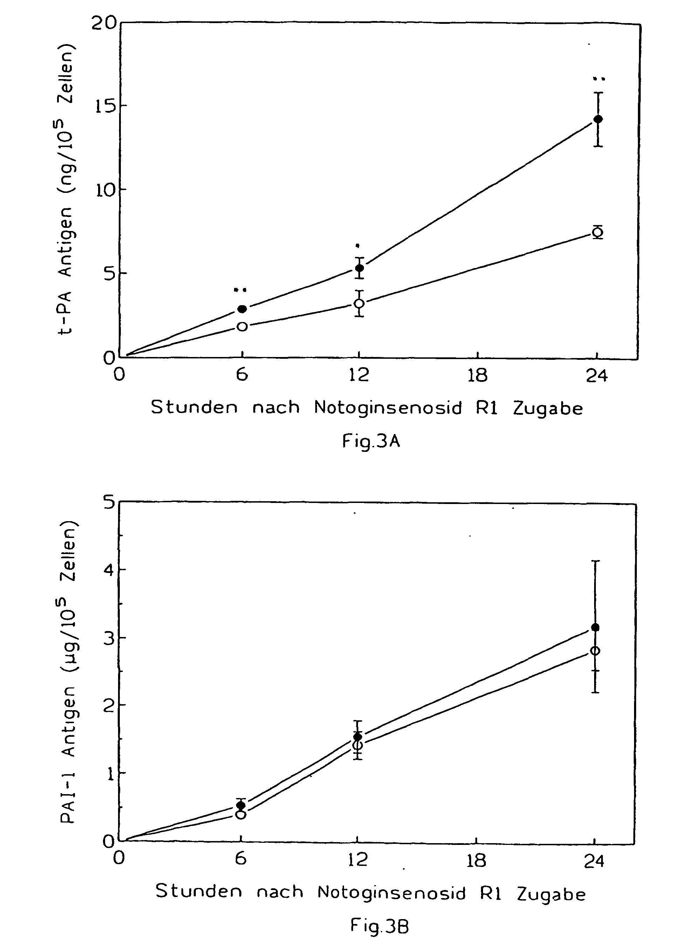

### Zeitverlauf der tPA Antigen (Feld A) und PAI-1 Antigen (Feld B) Produktion von kultivierten HUVECs nach Behandlung mit Notoginsenosid R1 (NR1): (Fig. 3)

HUVECs wurden für die angegebenen Zeiträume in Abwesenheit (offene Kreise) oder Gegenwart von 100µg/ml NR1 (volle Kreise) inkubiert. Zu den angegebenen Zeitpunkten wurde das entsprechende CM geerntet und wie unter Materialien und Methoden beschrieben auf tPA Antigen und PAI-1 Antigen hin getestet. Die Resultate stellen die Mittelwerte von 3, jeweils in 3-facher Anordnung ausgeführten Experimente dar. Die Werte sind als Mittelwerte ± S.D., *p<0,05; **p<0,01 im Vergleich zur Kontrolle angegeben. Wie in Fig. 3 gezeigt stieg das tPA Antigen im CM von HUVECs, die für 6, 12 oder 24 Stunden mit 100 µg/ml NR1 behandelt wurden im Vergleich zur Kontrolle zeitabhängig an, wo hingegen das PAI-1 Antigen im CM solcher Art behandelter Zellen sich nicht signifikant änderte.

### Notoginsenosid R1 beeinflußt die uPA Antigensekretion von kultivierten HUVECs

Wenn das CM von HUVECs, die in Gegenwart von 100 µg/ml NR1 inkubiert wurden auf uPA Antigen hin untersucht wurde, fand sich eine signifikante Änderung in der Menge des von diesen Zellen produzierten uPA Antigens im Vergleich zum CM von HUVECs, die unter Kontrollbedingungen kultiviert worden waren (100 µg/ml NR1: 2,9 ± 0,6 ng/106 Zellen/24h; Kontrolle: 2,5 ± 0,8 ng/106 Zellen/24h; n=9

### Beispiel 2: Effekt von NR1 auf die tPA und PAI-1 Aktivität in kultivierten humanen Umbilicalvenen Endothelzellen

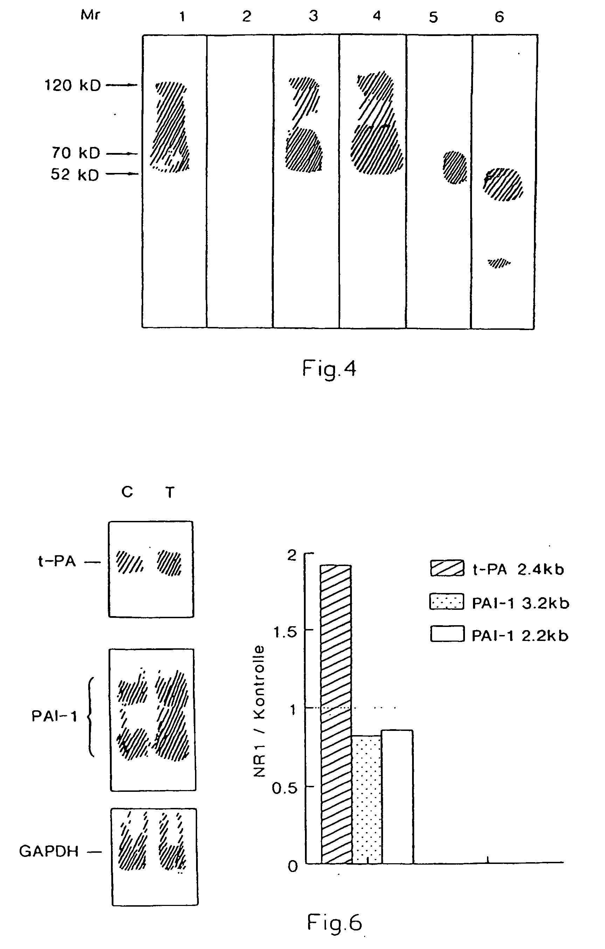

### Notoginsenosid R1 steigert die tPA Aktivität und reduziert die PAI-1 Aktivität in kultivierten HUVECs. Fibrinautographie (FA) von HUVECs CM Proben: (Fig. 4)

CM von HUVECs wurde nach 24 Stunden gesammelt und mit SDS PAGE und anschließender FA, wie unter Material und Methoden beschrieben untersucht. Bahn 1: unbehandeltes CM von HUVECs; Bahn 2: CM von HUVECs, das mit an Sepharose gekoppelten monoklonalen Anti-tPA Antikörpern vorinkubiert wurde; Bahn 3: CM von HUVECs, das mit an Sepharose gekoppelte monoklonalen Anti-uPA Antikörpern vorinkubiert wurde; Bahn 4: CM von HUVECs, das mit Sepharose 4B vorinkubiert wurde; Bahn 5: gereinigter humaner tPA; Bahn 6: gereinigter humaner uPA. Wenn von HUVECs, die für 24 Stunden unter Kontrollbedingungen inkubiert wurden, gewonnenes CM mit SDS PAGE und anschließender FA analysiert wurde, fanden sich 2 dominante Lysezonen mit einem offensichtlichen Molekulargewicht von 70.000 bzw. 120.000. Diese Lysezonen konnten durch Präinkubation mit monoklonalen Anti-tPA Antikörpern nicht aber durch Präinkubation mit monoklonalen Anti-uPA Antikörpern entfernt werden (Fig. 4). Daher läßt sich schlußfolgern, daß die Lysezone bei 70 kDa von freiem tPA verursacht wurde und die hochmolekulare Lysezone von tPA in Komplex mit PAI-1 stammte.

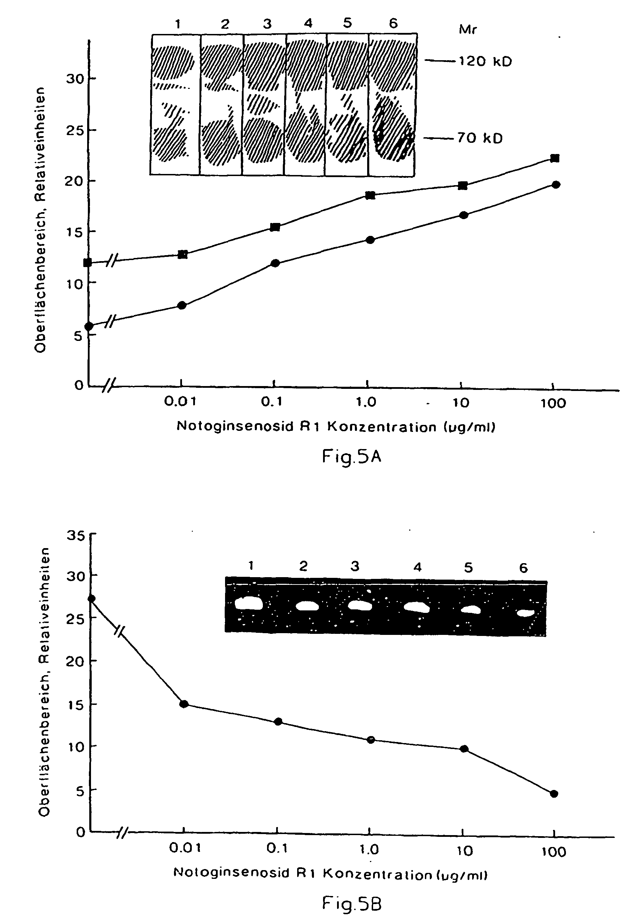

Effekt von Notoginsenosid R1(NR1) auf die tPA Aktivität und auf die mit dem tPA PAI-1 Komplex assozierte Aktivität (Feld A) und auf die PAI-1 Aktivität (Feld B) in kultivierten HUVECs nach Analyse mit Fibrinautographie (FA) und reverser Fibrinautographie (RFA): (Fig. 5)

Nach der Inkubation konfluenter HUVECs für 24 Stunden mit verschiedenen Konzentrationen von NR1 (0,001-100µg/ml) wurde das CM mit SDS PAGE und nachfolgender FA oder RFA wie unter Materialien und Methoden beschrieben, aufbereitet. Die Lysezonen und die lyseresistenten Zonen in diesen Fig. wurden auf Transparentpapier übertragen, ausgeschnitten und auf einer analytischen Waage gewogen. Die Gewichte dieser Transparentpapiere wurden gegen die Konzentrationen von NR1 aufgetragen (unteres Feld). Die Daten stellen Resultate eines von 3 unabhängigen Experimenten, die ähnliche Resultate zeigten, dar. Bahn 1: Kontrolle; Bahn 2: 0,01µg/ml NR1; Bahn 3: 0,1µg/ml NR1; Bahn 4: 1,0µg/ml NR1; Bahn 5: 10µg/ml NR1; Bahn 6: 100/ml NR1. Wenn das CM von HUVECs, die ohne oder mit steigenden Konzentrationen von NR1 für 24 Stunden kultiviert wurden, mit FA bzw. RFA analysiert wurde, konnte ein dosisabhängiger Anstieg in der Größe der Lysezonen festgestellt werden, wo hingegen die Größe der Lyse-resistenten Zonen mit steigenden Mengen von NR1 abnahmen (Fig. 5A und B). Wenn die Größe der Lysezonen bzw. der Lyse-resistenten Zonen wie in den Materialen und Methoden beschrieben quantifiziert wurde, konnte ein bis zu 3-facher Anstieg in der tPA-abhängigen Lyse festgestellt werden wo hingegen die PAI-1 abhängige Lyseresistenz auf 20% in Gegenwart von 100µg/ml NR1 im Vergleich zur Kontrolle abnahm (Fig. 5A und B).

### Beispiel 3: Effekt von NR1 auf die tPA und PAI-1 mRNA in kultivierten humanen Umbilicalvenen Endothelzellen

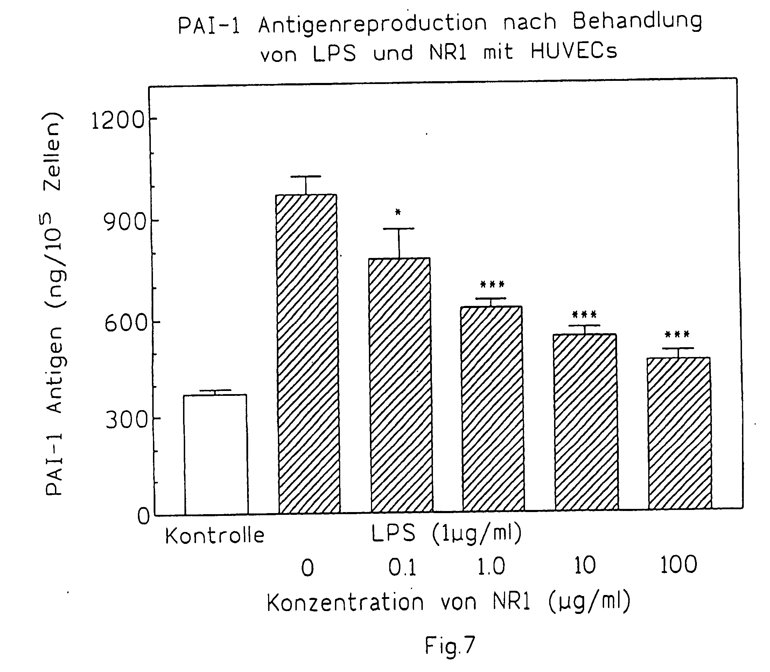

### Effekt von Notoginsenosid R1 (NR1) auf die tPA und PAI-1 mRNA Expression in HUVECs: (Fig. 6)

Konfluente HUVECs wurden für 12. Stunden in Abwesenheit (C) oder in Gegenwart (T) von NR1(100µg/ml) inkubiert. Die Northern Blot Analyse der RNA Extrakte von unbehandelten und NR1 behandelten HUVECs wurde unter Verwendung von 32P-markierten cDNA-Sonden für tPA, PAI-1 und GAPDH mRNA durchgeführt. Die Intensität der auf dem Autoradiogramm vorhandenen Banden wurde mit Hilfe der Densitometrie ausgewertet und die spezifische mRNA für tPA bzw. PAI-1 wurde gegen GAPDH mRNA normalisiert um Unterschiede in der Beladung zu berücksichtigen. Die Signalintensität wurde verglichen als das Verhältnis des Signals von mit NR1 behandelten HUVECs im Vergleich zum Signal von unbehandelten Kontrollzellen. Diese Daten stellen Resultate von 2 unabhängigen Experimenten, die ähnliche Ergebnisse zeigten dar. Wie in Fig. 6 gezeigt, war der stimulierende Effekt von NR1 auf die Sekretion von tPA in HUVECs auch auf dem Niveau der spezifischen mRNA Expression reflektiert. Die tPA spezifische mRNA stieg bis zu einem 2-fachen Wert in für 12 Stunden mit 100µg/ml NR1 behandelten HUVECs an, wo hingegen die PAI-1 spezifische mRNA Expression nicht durch NR1 reguliert wurde (3,2 kb: 82% der Kontrolle, 2,2kb: 86% der Kontrolle). Wenn Northern Blot Experimente in der Gegenwart von 10µg/ml Zycloheximid durchgeführt wurden, wurde der stimulierende Effekt von NR1 auf die tPA spezifische mRNA verhindert (Daten nicht gezeigt).

### Beispiel 4: Effekte von NR1 auf die durch Endotoxin verursachte Aufregulation von PAI-1 Antigen, Aktivität und PAI-1 mRNA in vitro.

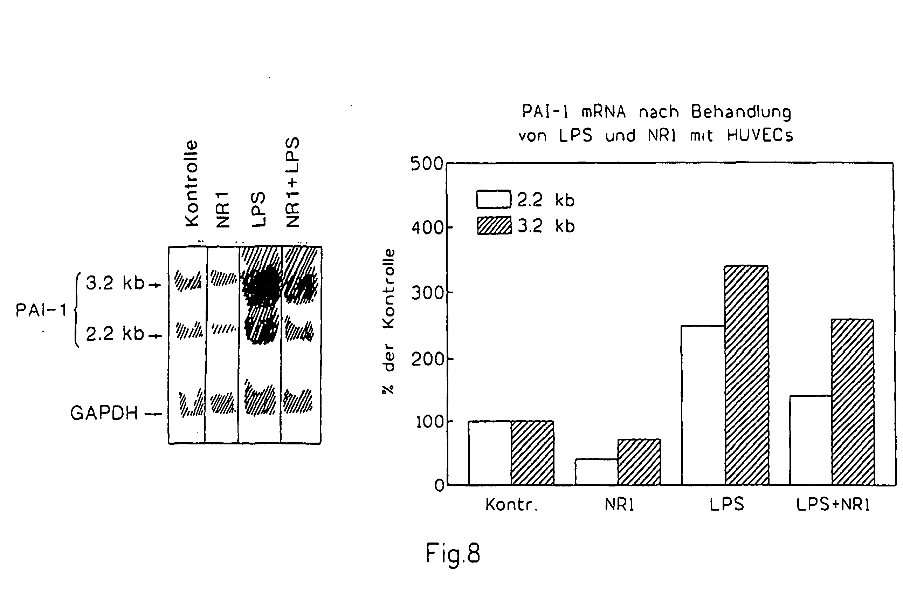

Wie in Fig. 7 gezeigt, wird die nach Behandlung der Zellen mit LPS (1µg/ml) für 12 Stunden erfolgte Aufregulation des PAI-1 Antigens durch gleichzeitige Behandlung mit verschiedenen Konzentrationen von NR1 antagonisiert. Das Ausmaß des Antagonismus war bezogen auf die NR1 Konzentration (0,1-100µg/ml) dosisabhängig und die durch LPS induzierte Zunahme des PAI-1 Antigens war durch die Koinkubation der Zellen mit 100µg/ml NR1 signifikant reduziert (Kontrollzellen: 347 ± 34ng/105 Zellen/12h, LPS behandelte Zellen: 946±42ng/105 Zellen/12h, LPS und NR1 behandelte Zellen: 469 ± 29ng/105Zellen/12h). Die Änderung der PAI-1 Aktivität der Zellen war parallel zur Änderung im PAI-1 Antigen (Kontrollzellen: 5,48 ± 0,78U/105Zellen/12h, LPS behandelte Zellen: 8,22±0,18U/105/Zellen/12h, LPS und NR1 behandelte Zellen: 4,77 ± 0,26U/105Zellen/12h, n=6). Die mRNA für PAI-1 wurde in Zellen, die mit einem µg/ml LPS und/oder 100µg/ml NR1 behandelt worden waren gemessen. Der durch LPS induzierte Anstieg zur zweifachen Menge an PAI-1 spezifischer mRNA (3,2kb) wurde in Gegenwart von sowohl LPS, als auch NR1 auf einem 1,37-fachen Anstieg reduziert (Fig. 8).

### Beispiel 5: Effekte von NR1 auf die durch LPS induzierte Aufregulation der PAI-1 Aktivität in vivo.

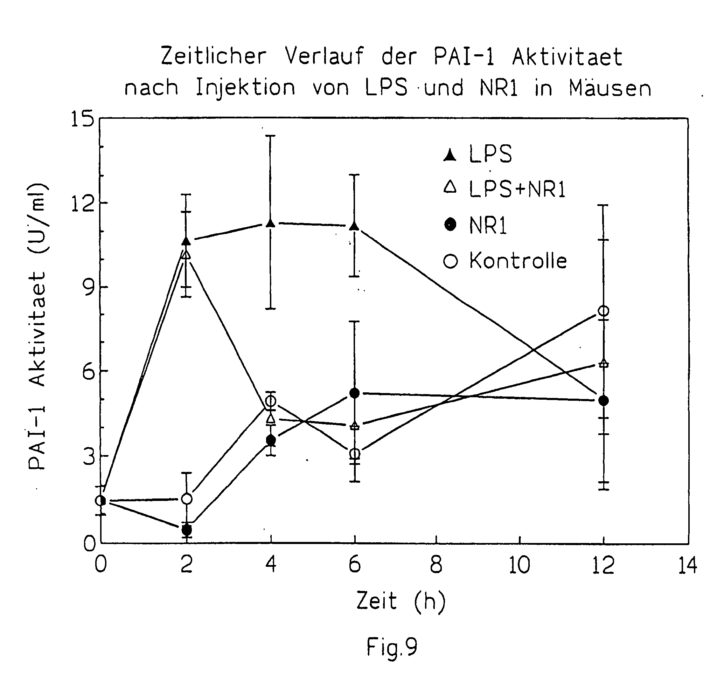

In vivo Studien zeigten, daß die Injektion von LPS in Mäusen in einem schnellen Anstieg der Plasma-PAI-1 Aktivität resultierte. Mit einer LPS Dosis von 10 ng/g (Körpergewicht) konnte ein signifikanter bis zu 7-facher Anstieg über den Kontrollwert 2 Stunden nach der Injektion festgestellt werden, während Höchstwerte 4 Stunden nach der Injektion erreicht wurden. Zu späteren Zeitpunkten kehrte die PAI-1 Aktivität graduell zu den Normalwerten zurück. Im Gegensatz dazu kehrte die PAI-1 Aktivität in gleichzeitig mit LPS und NR1 (1µg/g) behandelten Tieren 4 Stunden nach der Injektion zu den Kontrollwerten zurück (LPS behandelte Gruppe: 11,3 ± 3,1U/ml, LPS und NR1 behandelte Gruppe: 4,3 ± 1,0 U/ml, Kontrollgruppe 4,9 ± 0, 3U/ml, n=5-8) (Fig. 9).

### Beispiel 6: Effekte von NR1 auf die durch Endotoxin (LPS) bewirkte Induktion der TF Aktivität und mRNA in kultivierten HUVECs :

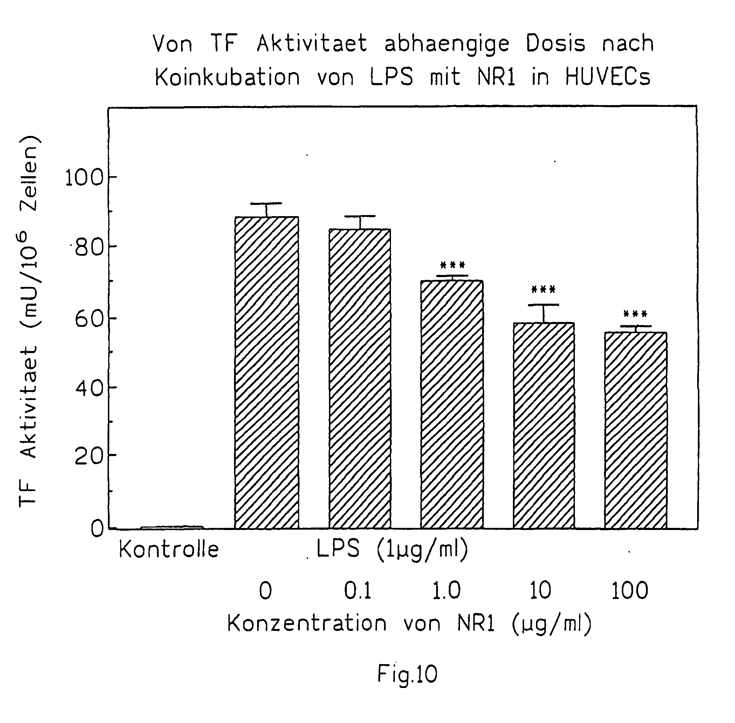

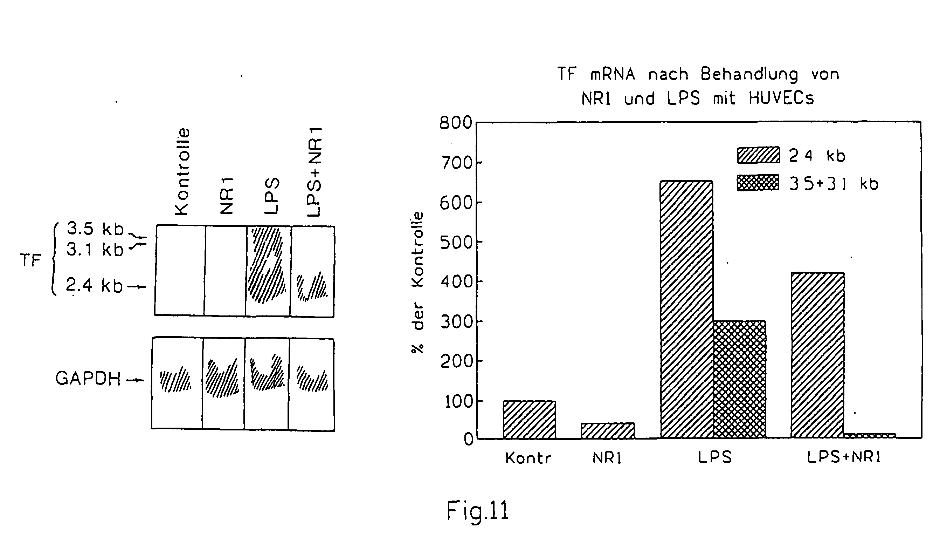

In unbehandelten HUVECs wurde nur eine sehr kleine Menge an TF Aktivität gefunden (0,78±0,15mU/106Zellen, n=9). Die TF-Aktivität stieg in HUVECs nach Behandlung mit LPS (1µg/ml für 6h) auf einen Wert von 88,6±6,5mU/106Zellen (n=6) an. Die nach 6 Stunden Behandlung mit 1µg/ml LPS gemessene TF-Aktivität in HUVECs wurde ebenfalls signifikant durch Koinkubation mit NR1 antagonisiert (LPS und NR1 behandelte Zellen: 56,0±1,9mU/106Zellen). Das Ausmaß des Antagonismus war bezogen auf die NR1 Konzentration ebenfalls dosisabhängig, wobei mit 100µg/ml MR1 eine etwa 36,8%ige Hemmung erreicht wurde (Fig. 10). Ein signifikanter Anstieg der TF mRNA wurde nach Behandlung von HUVECs mit LPS beobachtet, der eine 9-fache (2,4±3,1±3,5kb) Steigerung über Kontrollwerte nach 2 Stunden erreichte. Die durch LPS erhöhten TF mRNA Werte wurden in einer ähnlichen Weise durch Koinkubation mit NR1 antagonisiert; die TF mRNA wurde auf einen etwa 4-fach über der Kontrolle liegenden Wert reduziert. Die Behandlung der Zellen mit 100µg/ml NR1 alleine reduzierte die TF mRNA Werte auf 40% der Kontrollwerte (Fig. 11).

### Beispiel 7: Wirkung von Extrakten, welche Notoginseng R1 enthalten auf die fibrinolytische Aktivität beim Menschen.

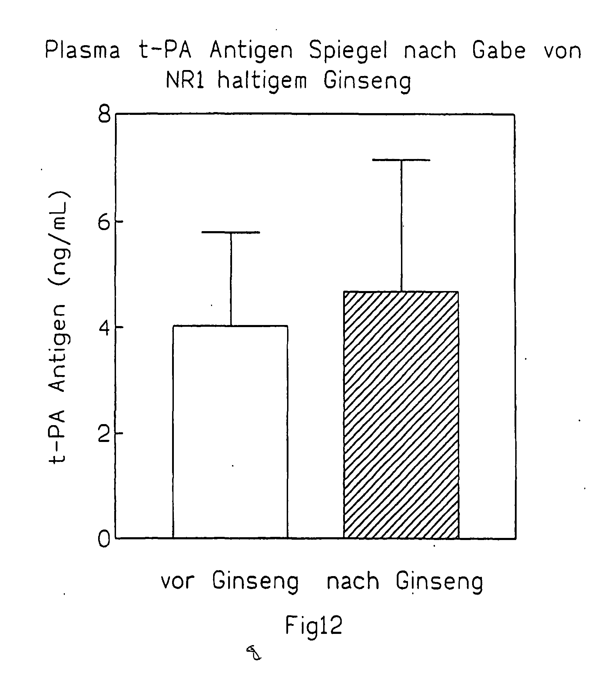

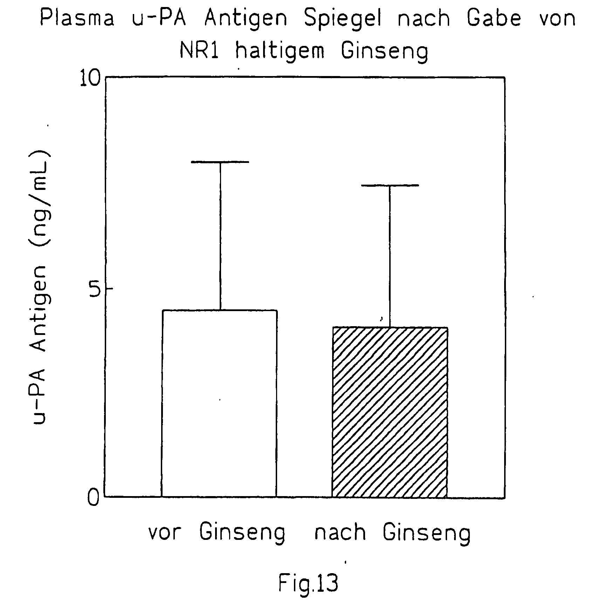

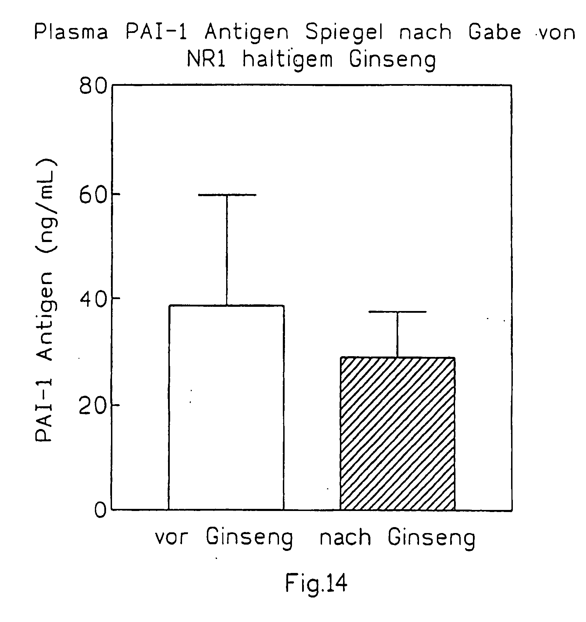

In Fig. 12, 13 und 14 sind die Effekte von NR1-enthaltenden Extrakten auf tPA, PAI und uPA bei gesunden Probanden angegeben. Es kommt zu einem Anstieg von tPA und einem Abfall von PAI-1, der den Effekten von NR-1 in der Gewebekultur entspricht.

## Patentansprüche

1. Verwendung von Notoginsenosid R1 (NR1) zur Herstellung eines Arzneimittels zur Behandlung von Endotoxin-Schock.

2. Verwendung von Notoginsenosid R1 (NR1) zur Herstellung eines Arzneimittels zur Behandlung von koronarer Herzerkrankung, peripherer Arterienerkrankung sowie Myocardinfarkt und Angina pectoris.

## Claims

1. The use of notoginsenoside R1 (NR1) for the manufacture of a drug for the treatment of endotoxin shock.

2. The use of notoginsenoside R1 (NR1) for the manufacture of a drug for the treatment of coronary heart disease, peripheral arterial disease as well as myocardism and angina pectoris.

## Revendications

1. Utilisation de la notoginsenoside R1 (NR1) pour la préparation d'un médicament pour le traitement d'un choc endotoxique.

2. Utilisation de la notoginsenoside R1 (NR1) pour la préparation d'un médicament pour le traitement d'une maladie cardiaque coronaire, d'une maladie des artères périphériques ainsi que de l'infarctus du myocarde et de l'angine de poitrine.
